(19) Europäisches Patentamt
European Patent Office
Office européen des brevets

(11) Veröffentlichungsnummer: **0 224 697**
**B1**

(12) # EUROPÄISCHE PATENTSCHRIFT

(45) Veröffentlichungstag der Patentschrift: **09.05.90**

(51) Int. Cl.$^5$: **A 01 N 53/00**

(21) Anmeldenummer: **86114455.8**

(22) Anmeldetag: **18.10.86**

(54) **Verwendung von synthetischen Pyrethroiden zur Herstellung von Mitteln zur Bekämpfung von Parasitosen bei Bienen.**

(30) Priorität: **31.10.85 DE 3538688**

(43) Veröffentlichungstag der Anmeldung:
**10.06.87 Patentblatt 87/24**

(45) Bekanntmachung des Hinweises auf die Patenterteilung:
**09.05.90 Patentblatt 90/19**

(84) Benannte Vertragsstaaten:
**AT BE CH DE ES FR GB GR IT LI NL SE**

(56) Entgegenhaltungen:
**EP-A-0 000 345**
**PESTICIDE SCIENCE, Band 16, Nr. 2, April 1985, Seite 205, Blackwell Scientific Publications, Londen, GB; S.W. SHIRES: "A step-wise evaluation of the effects of a new pyrethroid insecticide WL-85871 on honey-bees"**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Stendel, Wilhelm, Dr.**
**In den Birken 55**
**D-5600 Wuppertal 1 (DE)**
Erfinder: **Neuhauser, Hubert, Dr.**
**An der Jüch 59**
**D-5060 Bergisch-Gladbach 2 (DE)**
Erfinder: **Koeniger, Nikolaus, Dr.**
**Karl-von Frisch-Weg 1**
**D-6370 Oberursel (DE)**

(56) Entgegenhaltungen:
**PESTICIDE SCIENCE, Band 16, Nr. 2, April 1985, Seiten 206-207, Blackwell Scientific Publications, Londen, GB; L. GERIG: "Testing the toxicity of synthetic pyrethroid insecticides to bees"**
**PESTICIDE SCIENCE, Band 16, Nr. 4, August 1985, Seiten 409-415, Blackwell Scientific Publications, Londen, GB; J. DELABIE et al.: "Toxic and repellent effects of cypermethrin on the honeybee: Laboratory, glasshouse and field experiments"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 224 697 B1

**Beschreibung**

Die vorliegende Erfindung betrifft die verwendung von synthetischen Pyrethroiden zur Herstellung von Mitteln zur Bekämpfung von Parasitosen bei Honigbienen.

Bienenschädigende Parasiten sind bekannt. Insbesondere Milben schädigen zunehmend alle Entwicklungsstadien der Honigbiene. Ein besonderes Problem stellt dabei Varroa jacobsoni dar. Die durch sie hervorgerufenen Schädigungen sind inzwischen nahezu weltweit verbreitet. Befallene Bienenvölker gehen ohne geeignete Schutzmaßnahmen zugrunde.

Es ist bekannt, die Bekämpfung der Parasitosen durch Begasen der Bienenvölker mit Ameisensäure oder mit Brompropylat (4,4'-Dibrombenzilsäureisopropylester) durchzuführen. Es ist ferner bekannt, Behandlungen mit Coumaphos O,O-Di-ethyl-O-(3-chlor-4-methylcumarin-7-yl)-thionophosphorsäureester durchzuführen (DE—OS 33 41 017). Es ist auch bekannt geworden Pyrethrum-Extrakte zur Bekämpfung einzusetzen (Die Biene 2/1985, S.55). Diese Mittel sind zwar wirksam doch gleichzeitig bienentoxisch.

Es wurde nun gefunden, daß Parasitosen bei Honigbienen sehr erfolgreich durch synthetische Pyrethroide bekämpft werden können.

Synthetische Pyrethroide zählen zu den wirksamsten Insektiziden überhaupt. Sie sind wesentlich wirksamer als die aus Pflanzen gewonnenen Pyrethrum-Extrakte. Es war daher außerordentlich erstaunlich, daß man sie zur Behandlung von Parasitosen bei Honigbienen einsetzen konnte, ohne gleichzeitig die Honigbienen zu schädigen.

Zu den synthetischen Pyrethroiden zählen Verbindungen der Formel I

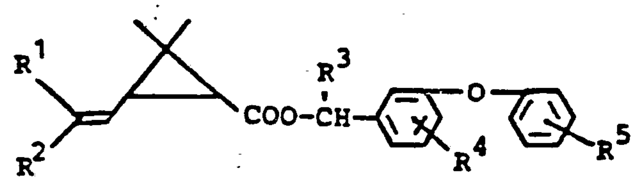

I

in welcher

$R^1$ für Halogen steht,

$R^2$ für Halogen, gegebenenfalls halogensubstituiertes Alkyl, gegebenenfalls halogensubstituiertes Phenyl steht,

$R^3$ für Wasserstoff oder CN steht,

$R^4$ für Wasserstoff oder Halogen steht,

$R^5$ für Wasserstoff oder Halogen steht.

Bevorzugt sind synthetische Pyrethroide der Formel I in welcher.

$R^1$ für Halogen, insbesondere Fluor, Chlor, Brom steht,

$R^2$ für Halogen, insbesondere Fluor, Chlor, Brom, Trihalogenmethyl, Phenyl, Halogenphenyl steht,

$R^3$ für Wasserstoff oder CN steht,

$R^4$ für Wasserstoff oder Fluor steht,

$R^5$ für Wasserstoff steht.

Bensonders bevorzugt sind synthetische Pyrethroide der Formel I in welcher

$R^1$ für Chlor steht,

$R^2$ für Chlor, Trifluormethyl, p-Chlorphenyl steht,

$R^3$ für CN steht,

$R^4$ für Wasserstoff oder Fluor steht,

$R^5$ für Wasserstoff steht.

Insbesondere seien Verbindungen der Formel I genannt in welcher

$R^1$ für Chlor steht,

$R^2$ für Chlor oder p-Chlorphenyl steht,

$R^3$ für CN steht,

$R^4$ für Fluor in 4-Stellung steht,

$R^5$ für Wasserstoff steht.

Im einzelnen seien folgende synthetische Pyrethroide genannt: Permethrin, Cypermethrin, Cyfluthrin, Cyhalothrin, Deltamethrin, Flumethrin.

Besonders hervorgehoben seien Cyfluthrin und Flumethrin, Insbesondere sei Flumethrin, 3-[2-(4-Chlorphenyl)-2-chlorvinyl]-2,2-dimethyl-cyclopropan-carbonsäure-α-cyano-3-phenoxy-4-fluor-benzylester, genannt.

Die Wirkstoffe liegen dabei in Form ihrer optischen und sterischen Isomeren, sowie in Form der Gemische dieser Isomeren vor.

Die Wirkstoffe sind bekannt (DE—OS 27 09 264, 27 30 515, 23 26 077, 27 38 150).

Parasitosen der Honigbiene sind solche, die durch folgende Parasiten hervorgerufen werden: Varroa jacobsoni, Acarapis woodi, Tropilaelaps clareae, Tropilaelaps koenigerum, Braula coeca, Galleria mellonella (Wachsmotte), Achroia grisella (kleine Wachsmotte).

Als Parasit sei besonders genannt Varroa jacobsoni. Der Parasit schädigt dabei direkt die Entwicklungsstadien der Bienen, auf denen er sich vermehrt. Er schädigt auch die erwachsenen Bienen. Er kann jedoch auch als Vektor für andere Bienenkrankheiten verantwortlich sein.

Die Behandlung der Honigbienen kann auf verschiedene Weise erfolgen;

1. durch direkten Kontakt mit dem Wirkstoff. Dazu wird dieser z.B. versprüht, verstäubt, verräuchert, verdampft, verdunstet oder in Trägerstoffe, die mit Bienen in Kontakt kommen, eingearbeiutet oder auf die Trägerstoffe aufgebracht,

2. durch den sozialen Futteraustausch im Bienenvolk und durch eine systemische Wirkung über die Hämolymphe der Biene. Dazu wird der Wirkstoff z.B. mit dem Futter oder Trinkwasser angeboten, oder in den Bienenstock gegossen oder gespritzt.

Die Behandlung kann prinzipiell ganzjährig erfolgen. Wird der Wirkstoff versprüht, verstäubt oder verräuchert, so ist es vorteilhat, die Behandlung in der brutarmen oder brutlosen Zeit durchzuführen, Wird der Wirkstoff verdampft, verdunstet, oder ist er in Trägerstoffe eingearbeitet, wird bevorzugt ganzjährig behandelt.

Im Fall 2 (oben) wird die Behandlung besonders vorteilhaft zum Zeitpunkt der Wintereinfütterung oder in der brutfreien Zeit durchgeführt.

Weiter kann das Bienenvolk als Kunstschwarm behandelt werden. Dies kann auch während der Brutperiode erfolgen.

Mittel, die versprüht werden, enthalten den Wirkstoff in Konzentrationen von 0,1—25 Gew.-%, bevorzugt von 0,3—10 Gew.-%.

Diese Mittel werden entweder direkt oder nach weiterem Verdünnen, bevorzugt mit Wasser, angewendet. Bei direkter Anwendung der Mittel ist die Ausbringung im ultra-low-volume-(ULV-)Verfahren mit dafür geeigneten üblichen Geräten bevorzugt. Die Mittel können auch unter Zuhilfenahme elektrostatischer Aufladung versprüht werden.

Die Mittel können vor der Anwendung auf Wirkstoff-Konzentrationen von $10^{-11}$—2 Gew.-%, bevorzgut $10^{-7}$—0,05 Gew.-%, verdünnt werden. Sie werden konventionell mit üblichen Geräten wie Rükenspritze, Kolbenpumpe, Lackierpistole versprüht.

Pro Volk werden je nach gewähltem Ausbringungsverfahren zwischen 1 µg—30 mg, bevorzugt 1 µg—10 mg Wirkstoff in Form der gegebenenfalls verdünnten Mittel eingesetzt.

Mit diesen Mitteln werden entweder die Bienen oder die Bienenwohnung oder Teile davon behandelt.

Die Mittel enthalten den Wirkstoff neben Verdünnungsmitteln und/oder Emulgatoren, die in den angewendeten Konzentrationen bienenverträglich sind.

Geeignete Verdünnungsmittel sind Alkohole, wie Methanol, Ethylalkohol, Tropanol, Isopropylalkohol, n-Butylalkohol, Amylalkohol, Octanol;

Glykole, wie Propylenglykol, 1,3-Butylenglykol, Ethylenglykol, Dipropylenglykolmonomethylether; Diethylenglycolmonomethylether;

Glycerin;

aromatische Alkohole wie Benzylalkohol;

Carbonsäureester, wie z.B. Ethylacetat, Benzylbenzoat, Butylacetat, Propylencarbonat, Milchsäureethylester;

aliphatische Kohlenwasserstoffe, Öle, wie z.B. Baumwollsaatöl, Erdnußöl, Maiskernöl, Olivenöl, Ricinusöl, Sesamöl;

Ketone, wie z.B. Aceton und Methylethylketon;

synth. Mono- und Triglyceride mit natürlichen Fettsäuren.

Weiterhin sind u.a. Verbindungen wie Dimethylsulfoxid, Dimethylacetamid, Dimethylformamid, N-Methylpyrolidon, Dioxan, 2-Dimethyl-4-oxymethyl-1,3-dioxalan gut als Verdünnungsmittel geeignet.

Besonders geeignet sind niedere Alkohole mit bis zu 8 Kohlenstoffatomen im Molekül, sowie niedere Ketone wie Methylthylketon und Ether des Ethylenglykols und des Propylenglykols.

Es können bei der Herstellung der erfindungsgemäß verwendbaren Mittel ein oder mehrere Verdünnungsmittel eingesetzt werden.

Geeignete Emulgatoren sind:

anionaktive Tenside, wie Na-Laurylsulfat, Fettalkohol-ethersulfate, Mono/Dialkylpolyglykoletherorthophosphorsäureester-Monoethanolaminsalz, Calciumalkylarylsulfonat,

kationaktive Tenside, wie Cetyltrimethylammoniumchlorid,

ampholytische Tenside, wie Di-Na-N-lauryl-:-iminodipropionat oder Lecithin,

nicht ionogene Tenside, z.B. polyoxyethyliertes Ricinusöl, polyoxyethyliertes Sorbitan-Monooleat, sorbitan-Monostearat, Glycerinmonostearat, Polyoxyethylenstearat, Alkylphenolpolyglykolether.

Bevorzugt seien als Emulgatoren genannt:

nicht-ionische, wasserlösliche Emulgatoren mit einem HLB (hydrophilic/lipophilic/balance-Wert) größer als 10, z.B. Emulgator NP 10® (Bayer AG), Alkylarylpolyglykolether; Renex 678® (Atlas Chemical Industries), Polyoxyethylenalkylarylether; Tween 40® (Atlas), Polyoxyethylensorbitanmonopalmitat; Myri 53® (Atlas), Polyoxyethylenstearat; Atlas G 3707®, Polyoxyethylenlaurylether; Atlas G 3920®, Polyoxyethylene oleylether; Atlas G9046 T®, Polyoxyethylenmannitanmonolaurat; Emulgator 1371 B® (Bayer AG), Alkylpolyglykolether; Emulgator 1736® (Bayer AG), Alkylpolyglykolether (Oleylpolyglykolether); Emulgator OX® (Bayer AG), Alkylpolyglykolether (Dodecylpolyglykolether); Ninox BM-2® (Stepan Chemical Co.),

ethoxyliertes Nonylphenol; Triton X-100® (Rohm and Haas Co.), Isooctylphenolpolyethoxyethanol; Cremophor EL®, polyoxyethyliertes Ricinusöl.

Die Emulgatoren sind in den erfindungsgemäßen Mitteln in Konzentrationen von 0—10-fachen, bevorzugt 0—5-fachen, des eingesetzten Wirkstoffs enthalten. Die Verdünnungsmittel werden jeweils zur gewünschten Endkonzentration ergänzt.

Die Mittel werden hergestellt durch Lösen des oder der Wirkstoffe im Lösungsmittel oder in einem Emulgator oder Emulgator-Lösungsmittelgemisch, falls notwendig, unter Erwärmung, Erforderlichenfalls erfolgt eine weitere Verdünnung der Mittel mit Wasser auf die gewünschte Konzentration.

Die Mittel zum Stäuben enthalten den Wirkstoff neben herkömmlichen bienenverträglichen Trägerstoffen, die zur Herstellung von Pudern oder wettable-powder geeignet sind.

Geeignete Trägerstoffe sind anorganische Trägerstoffe wie z.B. Talkum, Kaolin, Calciumcarbonat, Silikate, Bentonite, ferner organische Trägerstoffe wie z.B. Starken, z.B. Reisstärke, Zucker, Zellulose und Zellulosederivate.

Die Mittel werden gegebenenfalls unter Zusatz von Netzmitteln durch Vermischen des oder der Wirkstoffe mit den Trägerstoffen hergestellet. Geeignete Netzmittel sind z.B. die weiter oben angeführten Emulgatoren.

Mittel, die verräuchert werden, enthalten den Wirkstoff in Konzentrationen von $10^{-7}$—2 Gew.-% pro 100 g Trägermaterial. Als Trägermaterial dient das übliche Material für Rächerpräparate.

Mittel, durch die der Wirkstoff verdampft wird, sind z.B. Trägermaterialien, die mit wirkstoffhaltigen Mitteln imprägniert sind oder in die der Wirkstoff eingearteitet ist. Bevorzugt sind mit wirkstoffhaltigen Mitteln getränkte Papier-, Pappe-, Zellulose-, Stoff-, Filz-, Flies-, Lederplättchen oder -folien, die durch eine Wärmequelle erhitzt werden. Als Wärmequelle seien die für Verdampferplättchen üblichen elektrischen bzw. batteriebetriebenen Verdampferöfchen gennant.

Besonders vorteilhaft ist es, Mittel einzusetzen, in die der Wirkstoff eingearbeitet oder auf die er aufgebracht ist und die ohne zusätzliche Wärmequelle wirken. Damit läßt sich die Behandlung besonders einfach durchführen. Die Mittel werden einfach in den Bienenstock eingeführt. Das Volk erfährt keine Beunruhigung wie durch Sprühen, Räuchern oder Stäuben.

Die Behandlung wird durch Entfernung des Mittels beendet. Dies verhindert, daß die Milben ständig abnehmenden Wirkstoffkonzentrationen ausgesetzt sind. Dadurch wird einer Resistenzbildung bei den Milben vorgebeugt.

Die lang anhaltende Wirkstoffabgabe dieser Mittel erlaubt eine Langzeittherapie, die auch den Milbennachwuchs aus der Bienenbrut erfaßt.

In Abhängigkeit von Entwicklungszeitraum der Parasiten und der Bienen kann die Behandlung gezielt so erfolgen, daß entweder die gesamte Milbenbrut aus Arbeiterwaben oder die gesamte Milbenbrut aus Drohnenwaben erfaßt wird. Die Mittel bleiben dazu 12—90 bevorzugt 12—20 Tage im Bienenstock.

Es ist daher nicht erforderlich, in der brutfreien oder brutarmen Zeit zu behandeln oder das Volk vor der Behandlung künstlich brutfrei zu machen.

Erfolgt die Behandlung außerhalb der Honigernte, ist eine Kontaminierung des Honigs mit Wirkstoff nahezu ausgeschlossen.

Der Wirkstoff kann bei diesen Mitteln in Trägerstoffen enthalten oder eingearbeitet sein oder in geeigneter Form auf Trägerstoffen aufgebracht sein.

Trägerstoffe sind Formkörper, die am oder im Bienenstock angebracht werden. Auch Teile des Bienenstocks können aus Material geformt werden, in das der Wirkstoff eingearbeitet ist oder auf dessen Oberfläche der Wirkstoff aufgebracht ist oder das mit Wirkstoff getränkt oder imprägniert ist. Bevorzugt sind Schiede, die zwischen die Waben eingeschoben werden und die mit wirkstoffhaltigen Mitteln behandelt worden sind oder in die der Wirkstoff eingearbeitet ist.

Als Trägerstoffe können natürliche oder synthetische Trägerstoffe dienen. Natürliche Trägerstoffe sind z.B. Holz, Holzverarbeitungsprodukte, Pappe, Papier, Gummi, Kautschuk, Filz, Metall, Glas, Porzellan, keramische Materialien.

Synthetische Trägerstoffe zind z.B. Kunststoffe auf Basis Polyvinyl, PVC, Polyacrylat, Polymetacrylat, Epoxid, Polyurethan, Polyester, Polyamid, Cellulose und deren Arivate, Polyethylen, Polypropylen, synthetischer Kautschuk.

Als Trägerstoffe kommen aber auch Schichten in Frage, die auf einen festen oder flexiblen Untergrund aufgebracht sind. Solche Schichten können saugfähig sein und mit wirkstoffhaltigen Mitteln behandelt werden. Sie können aber auch nicht saugfähig sein und den Wirkstoff eingearbeitet enthalten. In der Regel handelt es sich bei diesen Schichten um haftfähige Polymere, denen gegebenenfalls inerte Füllsubstanzen zugesetzt werden. Als Polymere werden dabei die Lackrohstoffe der Farbenindustrie sowie z.B. Cellulosederivate, Acrylate und Metacrylate eingesetzt.

Als Beispiele für Füllstoffe zur Herstellung saugfähiger Schichten seien genannt: Kaolin, Calciumcarbonat, Silikate, Bentonite, Cellulose, Cellulosederivate, Stärke, Holzpulver. Der Wirkstoff ist dabei entweder bereits in schichtbildendes Material eingearbeitet oder die Schicht wird nachträglich z.B. mit dem oben beschriebenen zu versprühenden Mittel getränkt oder imprägniert oder besprüht.

Schichten, die den Wirkstoff eingearbeitet enthalten, können auch durch wirkstoffhaltige Anstriche oder Lacke gebildet werden. Diese enthalten den Wirkstoff in einer Konzentration von 0,00001—1, bevorzugt 0,001—10 Gewichtsprozent neben der üblichen Beschichtungsgrundmasse. Bevorzugt werden

Dispersionsanstriche und -lacke als Beschichtungsgrundmasse eingesetzt.

Schichten, die den Wirkstoff eingearbeitet enthalten, können aber auch Folien, Streifen, Bänder sein, die ein- oder mehrschichtig, sowie gegebenenfalls selbstklebend ausgebildet sind.

So kann eine wirkstoffhaltige Selbstklebefolie z.B. aus einer Klebeschicht, einer flexiblen Trägerschicht, einer wirkstoffhaltigen flexiblen Trägerschict, einer wirkstoffreien flexiblen Deckschicht bestehen. Die einzelnen Schichten bestehen aus an sich bekannten Polymermaterialien, die für die Herstellung solcher Schichten geeignet sind.

Wie bereits erwähnt, kann der Wirkstoff in diesen Formkörpern eingearbeitet enthalten sein. Diese enthalten den Wirkstoff in Konzentrationen von 0,00001—10 Gew.-%, bevorzugt 0,00001—1 Gew.-%, bezogen auf das Grundmaterial des Formkörpers.

Geeignete Formkörper sind Streifen, Bänder, Platten, aber auch, wue oben erwähnt, Bauteile des Bienenstockes.

Für die Herstellung der erfindungsgemäßen Formkörper können Polyvinylharze, Polyacrylate, Epoxyharze, Cellulose, Cellulosederivate, Polyamide und Polyester verwendet werden, die mit den obengenannten Wirkstoffen ausreichend verträglich sind. Die Polymeren müssen eine ausreichende Festigkeit und Biegsamkeit haben, um beim Formen nicht zu reißen oder brüchig zu werden. Sie müssen eine ausreichende Wanderung der Wirkstoffe and die Oberfläche des Formkörpers zulassen.

Typische Vinylharze sind beispielsweise Polyvinylhalogenide, wie Polyvinylchlorid, Polyvinylchlorid-Vinylacetat und Polyvinylfluorid; Polyacrylat- und Polymethacrylatester, wie Polymethylacrylat und Polymethylmethacrylat; und Polyvinylbenzole, wie Polystyrol und Polyvinyltoluol.

Für die Herstellung der erfindungsgemäßen Formkörper auf der Basis Polyvinylharz sind die Weichmacher geeignet, die üblicherweise zum Weichmachen von festen Vinylharzen verwendet werden. Der verwendete Weichmacher hängt von dem Harz und seiner Verträglichkeit mit dem Weichmacher ab. Geeignete Weichmacher sind beispielsweise Ester von Phosphorsäure, wie tricresylphosphat, Ester von Phthalsäure, wie Dimethylphthalat und Dioctylphthalat, und Ester von Adipinsäure, wie Diisobutyladipat. Es können auch andere Ester, wie die Ester von Azelainsäure, Maleinsäure, Ricinolsäure, Myristinsäure, Palmitinsäure, Ölsäure, Sebacinsäure, Stearinsäure und Trimellithsäure, sowie komplexe lineare Polyester, polymere Wiechmacher und epoxydierte Sojabohnenöle verwendet werden. Die Menge des Weichmachers beträgt etwa 10 bis 50 Gew.-%, vorzugsweise etwa 20 bis 45 Gew.-% der gesamten Zusammensetzung.

In den Formkörpern können noch weitere Bestandteile, wie Stabilisierungsmittel, Schmiermittel, Füllstoffe und Färbematerialien, enthalten sein, ohne daß dadurch die grundlegenden Eigenschaften der Zusammensetzung verändert werden. Geeignete Stabilisierungsmittel sind Antioxydationsmittel und Mittel, die den Formkörper vor ultravioletter Strahlung und unerwünschtem Abbau während der Bearbeitung, wie Strangpressen schützen. Einige Netzmittel wie epoxydierte Sojabohnenöle, dienen außerdem als sekunddäre Weichmacher. Als Schmiermittel können beispielsweise Stearate, Stearinsäure und Polyethylen mit niedrigem Molekulargewicht verwendet werden. Diese Bestandteile können in einer Konzentration bis zu etwa 20 Gew.-% der gesamten Zusammensetzung verwendet werden.

Bei der Herstellung der erfindungsgemäßen Formkörper auf Vinylsäurebasis weden die verschiedenen Bestandteile nach bekannten Mischverfahren trocken gemischt und nach bekannten Strangpreß- oder Spritzgußverfahren formgepreßt.

Die Wahl des Verarbeitungsverfahrens zur Herstellung der erfindungsgemäßen Formkörper richtet sich technisch grundsätzlich nach den rheologischen Eingenschaften des Formkörpermateriales und der Form des gewünschten Gebildes. Die Verarbeitungsverfahren können nach der Verarbeitungstechnologie oder nach der Art der Formgebung eingestellt werden. Bei der Verfahrenstechnologie kann man die Verfahren nach den bei ihnen durchlaufenen rheologischen Zuständen unterteilen. Danach kommen für viskose Formkörpermaterialien Gießen, Pressen, Spritzen und Auftragen und für elastoviskose Polymere Spritzgießen, Strangpressen (Extrudieren), Kalandrieren, Walzen und gegebenenfalls Kanten in Frage. Nach Art der Formgebung eingeteilt, lassen sich die erfindungsgemäßen Formkörper durch Gießen, Tauchen, Pressen, Spritzgießen, Extrudieren, Kalandrieren, Prägen, Biegen, Tiefziehen etc. herstellen.

Diese Verarbeitungsverfahren sind bekannt und bedürfen keiner näheren Erklärung. Im Prinzip gelten für Polymere wie Polyamide und Polyester die Erläuterungen, die oben beispielhaft für Polyvinylharze gemacht wurden.

Mittel die über den sozialen Futteraustausch im Bienenvolk wirken, sind z.B. Futterstoffe die den Wirkstoff enthalten. Als solche seien genannt: Zuckergranulate, zuckerhaltige Mischungen, Lösungen, Suspensionen oder Emulsionen. Diese enthalten den Wirkstoff in Konzentrationen von 0,5—20 Gew.-%, vorzugsweise von 1—10 Gew.-%. Diese Mischungen werden mit Wasser oder Zuckerlösung auf Anwendungskonzentrationen des Wirkstoffs von $10^{-8}$—1 Gew.-%, bevorzugt 0,0001—0,01 Gew.-%, besonders bevorzugt auf 0,0001—0,005 Gew.-% weiter verdünnt.

Ihre Herstellung ist z.B. beschrieben in DE—OS 3 341 017.

Beispiel A

Schiede (durchlöcherte Sperrholzplatten die im Bienenstock zwischen die Honigwaben eingehängt werden von 1553 $cm^2$) wurden pro Schied von jeder Seite mit 10 ml einer Lösung von 0,000239 g Flumethrin in 20 ml n-Hexan bestrichen. Die Schiede wurden 24 Stunden bei 34°C getrocknet und anschließand in Bienenstöcke gehängt, die Bienenvölker enthielten, die von Varroa jacobsoni befallen

waren. Pro Bienenstock wurden 2 behandelte Schiede eingehängt. Zur Kontrolle wurden unbehandelte Schiede verwendet. Am Ende des Versuchs wurde das gesamte Bienenvolk abgetötet, um eventuell überlebende Varroa zu erfassen. Es wurden folgende Ergebnisse erzielt:

| Volk-Nr. | Zahl der Bienen | Zahl der getöteten Varroa nach 1 Tag, | nach 2—12 Tagen | Zahl der überlebenden Varroa | Wirksamkeit |
|---|---|---|---|---|---|
| 1 | 13,700 | 172 | 29 | 4 | 98% |
| 2 | 14,410 | 170 | 9 | 0 | 100% |
| 3 | 9,900 | 230 | 18 | 1 | 99% |
| Kontrolle | | | | | |
| a | 12,500 | 1 | 13 | 273 | |
| b | 13,720 | 0 | 5 | 98 | |
| c | 8,910 | 2 | 7 | 431 | |

Beispiel B

Ca. 25 durch Varroa jacobsone verseuchte frisch aus einem Volk genommene Bienen wurden in einem Drahtkäfig durch Sprühen mit wäßrigen Wirkstoffkörpern unterschiedlicher Konzentrationen behandelt. Die Tiere erhielten eine wäßrige Zuckerlösung zur Nahrung. Es wurde gezählt, wieviele Bienen und wieviele Varroa während des Versuches (2 Tage) starben. Nach 2 Tagen wurden die Bienen abgetötet um noch überlebende Varroa zu erfassen. Es wurde die Mortalität der Varroa und der Bienen bestimmt.

| Wirkstoff | Konzentration | Mortalität in % Varroa | Bienen |
|---|---|---|---|
| Gemisch natürlicher Pyrethrine mit Tiperomybutoxid (Spruzit®) | 0,005 | 25 | — |
| | 0,01 | 58 | 19 |
| | 0,05 | 68 | 26 |
| | 0,08 | 67 | — |
| | 0,1 | 72 | 62 |
| | 0,5 | 100 | 100 |
| Kontrolle | | 23 | 5 |
| Flumethrin | 0,0001 | 100 | 6 |
| | 0,001 | 100 | 37 |
| | 0,01 | 100 | 86 |
| Kontrolle | | 7 | 5 |

Beispiel 1

| | | |
|---|---|---|
| Flumethrin | | 2 g |
| Emulgator Toximul R® | Mischung aus Alkylbenzolsulfonat-Ca und nichtionogenen Emulgatoren (HLB-Wert: >10) | 7 g |
| Emulgator Toximul S® | Mischung aus Alkylbenzolsulfont Ca und nichtionogenem Emulgator (HLB-Wert: >10) | 5 g |
| Solvesso 200® | (Alkylnaphthalin Gemisch aus hochsiedenden Erdölfraktionen) | ad 100 ml |

Beispiel 2

| | | |
|---|---|---|
| Flumethrin | | 1,6 g |
| Emulgator 368® | Alkylarylpolyglykolether (Molekulargewicht ca. 1165) | 9 g |
| Emulgator N P 10® | Nonylphenolpolyglykolether | 9 g |
| Dimethylformamid | | 10 g |
| Solvesso 200 | | ad 100 ml |

Beispiel 3

| | | |
|---|---|---|
| Flumethrin | | 0,5 g |
| Emulgator Atlox® | (Gemisch aus Polyoxyethylenether, Polyoxyglycerid, Alkylarylsulfonat — sehr leicht wasserlöslich) | 4 g |
| Emulgator Atlox 3404® | (Gemisch aus Polyoxyethylenalkylarylether, Alkylarylsulfonat — bildet Emulsion in Wasser) | 2 g |
| Emulgator Atlox 3409® | (Gemisch aus nichtionischen und anionischen Emulgatoren — löslich in Wasser) | 4 g |
| Solvent PC 2 | (hochsiedende aromatische Erdölfraktion) | ad 100 ml |

Beispiel 4

| | | |
|---|---|---|
| Flumethrin | | 0,1 g |
| Emulgator 368 | | 30 g |
| Dowanol DPM® | (Dipropylenglykolmethylether) | ad 100 ml |

Beispiel 5

| | |
|---|---|
| Flumethrin | 0,25 g |
| Emulgator 368 | 10 g |
| Emulgator NP 10 | 10 g |
| Solvesso 200 | 20 g |
| Dowanol DPM | ad 100 ml |

Beispiel 6

| | |
|---|---|
| Flumethrin | 0,2 g |
| Emulgator 368 | 9 g |
| Emulgator NP 10 | 9 g |
| Solvesso 200 | 16 g |
| Dowanol DPM | 45 g |
| Wasser | ad 100 ml |

Beispiel 7

| | |
|---|---|
| Flumethrin | 0,1 g |
| Emulgator 368 | 10 g |
| Emulgator NP 10 | 10 g |
| Solvesso 200 | 10 g |
| Dowanol DPM | ad 100 ml |

Beispiel 8

| | | |
|---|---|---|
| Flumethrin | | 0,25 g |
| Emulgator Tween 80® | (Sorbitanmonooleat mit HLB-Wert: 4,5) | 8 g |
| Emulgator Span 80® | (Sorbitanmonooleat mit H1B-Wert: 15) | 4 g |
| N-Methylpyrrolidon | | ad 100 ml |

Beispiel 9

| | | |
|---|---|---|
| Flumethrin | | 0,1 g |
| Cremophor HS 15® | (Polyoxyethylen-600-hydroxystearat) | 20 g |
| Chlorbenzol | | ad 100 ml |

Beispiel 10

| | | |
|---|---|---|
| Flumethrin | | 0,1 g |
| Emulgator W® | (Alkylarylpolyglykolether, Molekulargewicht ca. 853) | 90 g |
| Dimethylisosorbit | | ad 100 ml |

Beispiel 11

| | | |
|---|---|---|
| Wirkstoff: | Flumethrin | 0,5 g |
| Netzmittel: | Emulvin W® (Alkylarylpolyglykolether) | 3,0 g |
| | Wasser | ad 100 ml |

Beispiel 12

| | | |
|---|---|---|
| Wirkstoff: | Flumethrin | 5,0 g |
| Netzmittel: | wie Beispiel 1 | 30,0 g |
| | Wasser | ad 100 ml |

Beispiel 13

| | | |
|---|---|---|
| Wirkstoff: | Flumethrin | 2,0 g |
| Netzmittel: | NP 10® (Alkylarylpolyglykolether) | 40,0 g |
| | Wasser | ad 100 ml |

Beispiel 14

| | | |
|---|---|---|
| Wirkstoff: | Flumethrin | 5,0 g |
| Netzmittel: | Emulgator SZZ 14® | 20,0 g |
| | Wasser | 5,0 g |
| Lösungsmittel: | Isopropanol | ad 100 ml |

Beispiel 15

1 g Flumethrin wird mit 99 g Talkum gründlich vermischt. Von dieser Mischung werden 5 g mit 95 g Talkum gründlich vermischt.

Herstellung von Emulsion-Konzentraten

Beispiel 16

| | |
|---|---|
| Flumethrin 100% | 25,00 g |
| nichtionischer Emulgator (Emulgator 368®) | 25,00 g |
| Dipropylenglycolmonomethylether | ad 100 ml |

Die Substanzen werden zusammengewogen und so lange gerührt, bis eine klare Lösung entstanden ist.

Vor Gebrauch wird die Lösung auf ihre Anwendungskonzentration verdünnt.

Beispiel 17

| | |
|---|---|
| Flumethrin 100% | 1,00 g |
| Polyoxyethylenstearat | 0,50 g |
| Sorbitan-sesquivleat | 0,40 g |
| Wasser | 4,00 g |
| Polyacethylenglykol | ad 100 ml |

Herstellung und Anwendung wie bei Beispiel 16.

Herstellung von Verdampferplättchen :

Beispiel 18

20 mg Flumethrin werden in 100 ml Lösungsmittel Ethanol gelöst.

Mit 0,5 ml dieser Lösung wird ein entsprechendes handelsübliches Filterkarton-Plättchen getränkt. (Die Größe des Plättchens hängt von der ausgewählten Verdampfer-Einrichtung ab). Diese Plättchen werden durch Trocknen von Lösungsmittel befreit und enthalten 0,1 mg Flumethrin pro Plättchen.

Herstellung eines PVC-Formkörpers :

Beispiel 19

| | |
|---|---|
| Flumethrin | 0,5 g |
| Isobutyladipat | 15,5 g |
| Dialkylpythalat | 8,0 g |
| Polyoxyethyliertes Rizinusöl | 2,0 g |
| Stearinsäure | 0,8 g |
| Farbstoff | 0,1 g |
| Polyvinylchlorid | 73,1 g |
| | 100,0 g |

100,0 kg dieser Mischung werden in einem Mischer — in der üblichen Arbeitsweise für Weich-PVC — homogen gemischt.

Diese Mischung wird auf einer Spritzgußmaschine zu einem Wabenschied verarbeitet. Gewicht des Schieds: 86,0 g.

Obige Mischung wird statt mit 0,5 mit 0,25 g Wirkstoff hergestellt und auf einer entsprechenden Kalandrier-Vorrichtung zu einer Folie VM der Größe eines DIN-A4-Blattes ausgewalzt. Gewicht der Folie 50,0 g. Das Blatt wird in den Bienenstock gelegt.

Herstellung eines beschichteten Trägers

Beispeil 20

Auf einer Folie aus 2 mm dickem Polyethylen wird eine Lösung von Flumethrin in Emulgator Span 20® Atlas und Ethanol mittels einer Rakel gleichmäßig aufgetragen. Die Lösung wird so eingestellt, daß 1 mg Flumethrin pro 100 $cm^2$ Oberfläche und 0,5 mg Emulgator pro 100 $cm^2$ aufgetragen werden. Das Lösungsmittel wird abgedampft und die Folie in einer Form ausgestanzt, die sich in den Bienenstock einfach einpassen läßt.

Herstellung eines getränkten Trägers mit Polymer-(=Lack)-Zusatz

Beispiel 21

Mit Kieselgur beschichtete Aluminiumfolien werden mit einer Lösung aus Flumethrin und Polyvinylalkohol so behandelt, daß nach dem Trocknen auf der Folie pro 100 $cm^2$ 5 mg Flumethrin und 20 mg Polyvinylalkohol zurückbleiben.

Die Trägerformen der letzten beiden Beispiele können mit einem Haftkleber versehen sein. Nach Abziehen des Klebschutzes lassen sie sich in leere Schiede einfach einkleben.

Herstellung eines Granulates zur Anwendung beim sozialen Futteraustausch

Beispiel 22

0,5 kg Flumethrin werden in 7.5 l Ethanol unter vorsichtigem Erwärmen gelöst und in einem Mischgranulator auf 99,5 kg Zucker gegossen während der Mischer läuft. Der vom Alkohol feuchte, gleichmäßig getränkte. Zucker wird getrocknet und gegebenenfalls gesiebt. 1,0 g des Granulates wird vor Gebrauch in einer Zuckerlösung aufgelöst, die den Bienen zur Nahrung dient.

**Patentansprüche**

1. Verwendung von synthetischen Pyrethroiden zur Herstellung von Mitteln zur Bekämpfung von Parasitosen bei Honigbienen.

2. Verwendung gemäß Anspruch 1 von synthetischen Pyrethroiden zur Herstellung von Mitteln zur Bekämpfung von Parasitosen bei Honigbienen, die aus Formkörpern bestehen, in denen synthetische Pyrethroide enthalten oder eingearbeitet oder auf sie aufgebracht sind und die im oder am Bienenstock angebracht werden.

3. Verwendung gemäß Anspruch 1 und 2 von synthetischen Pyrethroiden zur Herstellung von Mitteln zur Bekämpfung von Varroa jacobsoni auf Honigbienen.

4. Verwendung gemäß Ansprüche 1 bis 3 von synthetischen Pyrethroiden zur Herstellung von Mitteln zur Bekämpfung von Parasitosen bei Honigbienen, die 0,3—10 Gewichtsprozent des Wirkstoffes neben Verdünnungsmitteln und/oder Emulgatoren, die in den angewendeten Konzentrationen bienenverträglich sind, enthalten.

5. Verwendung gemäß Ansprüche 1—3 von 0,00001—1 Gewichtsprozent synthetischer Pyrethroide bezogen auf das Grundmaterial zur Herstellung von Formkörpern zur Bekämpfung von Parasitosen bei Honigbienen, in die der Wirkstoff eingearbeitet ist oder in denen er enthalten ist und die im oder am Bienenstock angebracht werden.

6. Verwendung gemäß Ansprüche 1, 3 und 4 von synthetischen Pyrethroiden zur Herstellung von Mitteln zur Bekämpfung von Parasitosen bei Honigbienen, die den Wirkstoff neben Verdünnungsmitteln und/oder Emulgatoren enthalten und die in den Bienenstock gegossen oder gespritzt werden.

7. Verwendung gemäß Ansprüchen 1—6 von 3-[2-(4-Chlorphenyl)-2-chlorvinyl]-2,2-dimethyl-cyclopropancarbonsäure- α-cyano-3-phenoxy-4-fluor-benzylester (Flumethrin) zur Herstellung von Mitteln zur Bekämpfung von Parasitosen der Honigbienen.

**Revendications**

1. Utilisation de pyréthroïdes synthétiques pour la préparation de produits servant à combattre les parasitoses des abeilles.

2. Utilisation selon la revendication 1 de pyréthroïdes synthétiques pour la préparation de produits servant à combattre les parasitoses des abeilles, produits qui consistent en objects moulés contenant les pyréthroïdes synthétiques ou dans lesquels on an incorporé les pyréthroïdes synthétiques ou sur lesquels on a appliqué les pyréthroïdes synthétiques et qui sont placés dans ou sur la ruche.

3. Utilisation selon les revendications 1 à 2 de pyréthroïdes synthétiques pour la préparation de produits servant à combattre Varroa jacobsoni infestant les abeilles.

4. Utilisation selon les revendications 1 à 3 de pyréthroïdes synthétiques pour la préparation de produits servant à combattre les parasitoses des abeilles, produits qui contiennent de 0,3 à 10% en poids de la substance active avec des diluants et/ou des agents émulsionnants tolérés par les abeilles aux concentrations observées.

5. Utilisation selon les revendications 1 à 3 de 0,00001 à 1% en poids de pyréthroïdes synthétiques par rapport à la matière de base servant à la préparation d'objets moulés conçus pour combattre les parasitoses des abeilles, objets dans lesquels on a incorporé la substance active ou qui contiennent la substance active et qui sont disposés dans ou sur la ruche.

6. Utilisation selon les revendications 1, 3 et 4 de pyréthroïdes synthétiques pour la préparation de produits servant à combattre les parasitoses des abeilles, produits qui contiennent la substance active avec des diluants et/ou des agents émulsionnants et qui sont coulés ou injectés dans la ruche.

7. Utilisation selon les revendications 1 à 6 du 3-[2-(4-chlorophényl)-2-chlorovinyl]-2,2-diméthyl-cyclopropane-carboxylate d'alpha-cyano-3-phénoxy-4-fluoro-benzyle (Flumethrin) pour la préparation de produits servant à combattre les parasitoses des abeilles.

**Claims**

1. Use of synthetic pyrethroids for the preparation of agents for controlling parasitoses in honey bees.

2. Use according to Claim 1 of synthetic pyrethroids for the preparation of agents for controlling parasitoses in honey bees, which consist of shaped articles in which synthetic pyrethroids are contained or incorporated or applied to them, and which are placed in or on the beehive.

3. Use according to Claim 1 and 2 of synthetic pyrethroids for the preparation of agents for controlling Varroa jacobsoni on honey bees.

4. Use according to Claims 1 to 3 of synthetic pyrethroids for the preparation of agents for controlling parasitoses in honey bees, which contain 0.3—10 per cent by weight of the active compound in addition to diluents and/or emulsifiers which are tolerated by the bees in the concentrations used.

5. Use according to Claims 1—3 of 0.00001—1 per cent by weight of synthetic pyrethroids based on the basic material for the preparation of shaped articles for controlling parasitoses in honey bees, into which the active compound is incorporated or in which it is contained and which are placed in or on the beehive.

6. Use according to Claims 1, 3 and 4 of synthetic pyrethroids for the preparation of agents for controlling parasitoses in honey bees, which contain the active compound in addition to diluents and/or emulsifiers and which are poured or sprayed into the beehive.

7. Use according to Claims 1—6 of α-cyano-3-phenoxy-4-fluoro-benzyl 3-[2-(4-chlorophenyl)-2-chlorovinyl]-2,2-dimethyl-cylcopropanecarboxylate (flumethrin) for the preparation of agents for controlling parasitoses of honey bees.